# EUROPEAN PATENT APPLICATION

(11) **EP 1 891 943 A2**
(43) Date of publication of application: **27.02.2008**
(21) Application number: 06778444.7
(22) Date of filing: 01.06.2006
(51) Int. Cl.: A61K 9/51, A61K 47/40, A61K 47/36

(54) **NANOPARTICLES COMPRISING CHITOSAN AND CYCLODEXTRIN**

(30) Priority: 02.06.2005 ES 200501331
(71) Applicant: UNIVERSIDADE DE SANTIAGO DE COMPOSTELA, 15782 Santiago de Compostela (ES)
(72) Inventor: ALONSO FERNÁNDEZ, Mª José, E-15782 Santiago de Compostela (ES); GARCÍA FUENTES, Marcos, E-15782 Santiago de Compostela (ES); MAESTRELLI, Francesca, E-15782 Santiago de Compostela (ES); MURA, Paola, E-15782 Santiago de Compostela (ES)
(74) Representative: ABG Patentes, S.L.
(86) International application number: PCT/ES2006/000322
(87) International publication number: WO 2006/128937

(57) **Abstract**

This invention relates to a system comprising nanoparticles designed for the release of biologically active molecules, where the nanoparticles comprise a) at least 40% by weight of chitosan or a derivative thereof and b) less than 60% by weight of a cyclodextrin or a derivative thereof, where both components a) and b) are mixed, without any covalent bond between them. This system allows for the efficient association of biologically active molecules, as well as their subsequent release in a suitable biological environment.

## Description

### FIELD OF THE INVENTION

This invention relates to nanoparticle systems designed for the release of biologically active molecules. Specifically, it relates to nanoparticle systems composed of a mixture of the polymer chitosan and a cyclodextrin, wherein a biologically active molecule may be located, as well as to methods for the obtainment thereof.

### BACKGROUND OF THE INVENTION

Polymeric nanoparticles are receiving special attention due to their prospects for improving the stability and promoting the transport and controlled release of drugs to certain regions of the body, overcoming the problems associated with the limited permeability of epithelial barriers. Amongst biodegradable polymers, chitosan has received great attention in recent years due to its properties as a mucoadhesive agent (C.-M. Lehr, J. A. Bouwstra, E. H. Schacht, and H. E. Junginger, Int. J. Pharm., 1992, 78, 43-48) and an absorption promoter (P. Artursson, T. Lindmark, S. S. Davis, and L. Illum, Pharm. Res., 1994, 11, 1358-1361). Furthermore, scientific studies endorse chitosan as a material with an acceptable toxicological profile (S. B. Rao and C. P. Sharma, J. Biomed. Mater. Res., 1997, 34, 21-28) which has already been approved by the FDA as an additive in animal nutrition (J. D. McCurdy, Advances in Chitin and Chitosan, Elsevier Applied Science, London, 1992, pp. 757-764). Chitosan [α(1-4) 2-amino-2-deoxy-β-D-Glucan] is a natural polysaccharide resulting from the deacetylation of chitin. However, in practise, the chitosans used as a nutritional supplement or for medical applications are random polymers of acetylated and deacetylated monomers.

Nanoparticles of chitosan have been widely studied as carriers for the transmucosal administration of a large number of therapeutic molecules (A. M. De Campos, Y. Diebold, E. L. Carvalho, A. Sanchez, and M. J. Alonso, Pharm. Res., 2004, 21, 803-810; R. Fernandez-Urrusuno, P. Calvo, C. Remuñán-López, J. L. Vila-Jato, and M. J. Alonso, Pharm. Res., 1999, 16, 1576-1581; A. Prokov, E. Kozlov, G. W. Newman, and M. J. Newman, Biotechnology and Bioengineering, 2002, 78, 459-466; A. Vila, A. Sanchez, K. Janes, I. Behrens, T. Kissel, J. L. Vila-Jato, and M. J. Alonso, Eur. J. Pharm. Biopharm., 2004, 57, 123-131). A noteworthy characteristic of these particle systems is their capacity to improve the absorption characteristics of low-permeability molecules (R. Fernandez-Urrusuno, P. Calvo, C. Remuñán-López, J. L. Vila-Jato, and M. J. Alonso, Pharm. Res., 1999, 16, 1576-1581; A. Vila, A. Sanchez, K. Janes, I. Behrens, T. Kissel, J. L. Vila-Jato, and M. J. Alonso, Eur. J. Pharm. Biopharm., 2004, 57, 123-131). Although nanoparticles of chitosan have proven capable of effectively associating with hydrophilic drugs, these systems usually present limitations for the association of hydrophobic drugs and, particularly, those with a low aqueous solubility. At present, only one reference has been found regarding the use of nanoparticles of chitosan with a very low-solubility drug (A. M. De Campos, A. Sanchez, and M. J. Alonso, Int. J. Pharm., 2001, 224, 159-168), although, in this study, a method of preparation requiring the use of organic solvents was necessary.

On the other hand, cyclodextrins are known as complexing agents for low-solubility molecules and as carriers for the administration of active principles. Amongst them, chemically modified cyclodextrins are currently the most widely used in pharmaceutical technology due to their greater chemical versatility. For example, the substitution of the hydroxyls by methyl, hydroxypropyl or carboxymethyl groups gives the molecules greater water solubility and better toxicity characteristics. Other cyclodextrins make it possible to endow complexes with limited solubility (used in the formulation of sustained-release systems) or temperature-dependent solubility.

Recently, other potential utilities of cyclodextrins as pharmaceutical excipients have been demonstrated. Thus, complexation in cyclodextrins has proven capable of reducing the degradation kinetics of certain labile drugs, or the tendency to form inactive peptide aggregates, such as insulin. Furthermore, it has been shown that certain cyclodextrins have the capacity to promote the absorption of drugs due to the fact that they produce slight destructurings in the cell membranes as a result of complexation of the lipids thereof.

There are various documents which disclose the joint use of cyclodextrins and chitosan as a polymer in solution, gels or as solid macroscopic matrices (US2002150616, US5476654, US5330764, US6677346, US6497901, US5849327). US patent application US2002150616 proposes a mixture consisting of a low-solubility drug, a cyclodextrin and a hydrophilic polymer. EP0730869 discloses drug release systems also composed of mixtures of polymers and cyclodextrins.

Documents US5843347, US5840341 and US5639473 disclose polymer compositions in solution, in macroscopic particles or microparticles. The methods described for the formation of particles, such as extrusion (US5843347) or the formation of water-in-oil emulsions (US5639473), do not make it possible to produce particles smaller than several micrometres.

WO9961062 relates to the preparation of polymeric microparticles with cyclodextrins, where the cyclodextrins have the function of protecting the drug from potentially unfavourable interactions with the polymer matrix. Patent US6630169 discloses the formation of microstructures as vaccine carriers by transmucosal routes.

Patent US5639473 relates to the modification of hydrophilic polymers (such as chitosan) or oligosaccharides (such as cyclodextrins) by crosslinking with disulphur groups. According to the description of said invention, the proposed method leads to particle systems of between 0.1 and 20 micrometres.

WO03027169 discloses the formation of hydrophilic polymer derivatives with covalently bound cyclodextrins and their utility for the formation of pharmaceutical systems (including micro- and nanoparticles).

Patent US619757 discloses a method of preparation which includes the crosslinking in emulsion of the poly- or oligosaccharides composing the matrix to produce ether-type bonds between these molecules.

Patents US5700459 and US6649192 disclose methods for the formation of nanoparticles of chitosan for pharmaceutical applications. In both patents, the nanoparticles are formed by interaction of a polycation (such as chitosan) with a polyanion (such as tripolyphosphate). US5700459 mentions the possible use of certain cyclodextrins (aminocyclodextrins) as a substitute material for another potential polycation such as chitosan.

WO9704747 proposes the encapsulation of drugs or drug-cyclodextrin complexes in nanometric hydrogel matrices which may subsequently be coated with liposomes and/or mucoadhesion adjuvants. The proposed method requires precipitation of the polymer from an organic phase into an aqueous phase, and the drugs containing cyclodextrin are added in the aqueous phase wherein the polymer precipitates, and not jointly therewith. This factor in the method may lead to poorly efficient encapsulations of certain drugs.

It is worth highlighting that the microencapsulation techniques designed for the formation of microparticles generally differ from the nanotechnologies designed for the formation of nanoparticles. WO 9804244 discloses the formation of nanoparticles of chitosan.

### BRIEF DESCRIPTION OF THE INVENTION

The inventors have found that a system composed of nanoparticles of chitosan and a cyclodextrin allows for an efficient association of biologically active molecules, as well as their subsequent release in a suitable biological environment. These nanoparticles exhibit an improved capacity for encapsulating or associating hydrophobic drugs as compared to nanoparticles of chitosan without cyclodextrin. Furthermore, the cyclodextrins contribute new characteristics to the nanoparticle system, such as better protection of the associated biologically active molecule and a greater capacity to promote absorption, especially for those low-permeability molecules. Thus, in vivo studies have proven the capacity of the system of the invention to transport low-permeability drugs through the epithelial barriers, by interacting with the nasal mucous membrane, additionally crossing the nasal epithelium.

An additional characteristic exhibited by the nanoparticles present in the system of the invention is their high stability in cell culture mediums and, more significantly, in simulated intestinal fluids, where it has been shown that the physicochemical properties of the nanoparticles do not vary for at least four hours. This property makes these systems suitable for use by different administration routes and, in particular, for oral administration, allowing for drug release in the suitable biological environment. Moreover, release studies with different drugs have demonstrated that the nanoparticles make it possible to release the active principle at a controlled, gradual rate.

On the other hand, the possibility to incorporate and release nucleic acid-based macromolecules, such as DNA plasmids, has made it possible to observe, by means of *in vitro* studies, the nanoparticles' capacity to transfect cell cultures in a very efficient manner, which makes the system of the invention potentially suitable for use in gene therapy.

Thus, one object of this invention relates to a system comprising nanoparticles designed for the release of a biologically active molecule, where the nanoparticles comprise a) at least 40% by weight of chitosan or a derivative thereof and b) less than 60% by weight of a cyclodextrin or a derivative thereof, where both components a) and b) are mixed without covalent bonds.

Optionally, the nanoparticles may additionally comprise an ionic crosslinking agent which allows for the gelation of chitosan in the form of nanometric structures.

A second aspect of the present invention relates to a nanoparticle system such as that defined above which, in addition, comprises a biologically active molecule.

In another aspect, the invention relates to a pharmaceutical composition which comprises a nanoparticle system such as that defined above and a biologically active molecule capable of preventing, palliating or curing diseases. Moreover, one may find peptides, proteins or polysaccharides, which are not considered to be active biological molecules "per se" but may contribute to the efficacy of the administration system, trapped within the nanostructure.

Another aspect of the invention is a vaccine which comprises a nanoparticle system such as that defined above and an antigen. In a preferable aspect, the composition or vaccine is designed for transmucosal administration.

In another aspect, the invention relates to a cosmetic composition which comprises a nanoparticle system such as that described above.

Another aspect of the invention is a method for the obtainment of a system designed for the release of a biologically active molecule such as that defined, which comprises:
a. preparation of a solution of chitosan or a derivative thereof in an aqueous medium or in a mixture of water with a polar solvent;
b. preparation of a solution of a cyclodextrin or a derivative thereof in an aqueous medium or in a mixture of water with a polar solvent and, optionally, a crosslinking agent; and
c. mixing, under stirring, of the solutions of steps a) and b) in such a way that the nanoparticles of chitosan-cyclodextrin are spontaneously obtained.
or, optionally:
a. preparation of a solution of chitosan or a derivative thereof and a cyclodextrin or a derivative thereof in an aqueous medium or in a mixture of water with a polar solvent;
b. preparation of a solution of the crosslinking agent in an aqueous medium or in a mixture of water with a polar solvent;
c. mixing, under stirring, of the solutions of steps a) and b) in such a way that the nanoparticles of chitosan-cyclodextrin are spontaneously obtained.

The biologically active molecule may be directly incorporated to the solutions of steps a) or b); however, in a variant of the method the active molecule may be dissolved prior to the addition to steps a) or b) in an aqueous medium or in a mixture of water with a polar solvent.

A final aspect of the invention relates to the use of a system such as that described above for the preparation of a gene therapy drug.

### DETAILED DESCRIPTION OF THE FIGURES

- **Figure 1:**: TEM images of chitosan-(hydroxypropyl-β-cyclodextrin) formulations. Formulations prepared with 25 mM hydroxypropyl-β-cyclodextrin and 2 mg/ml of tripolyphosphate (left-hand-side image) or 1.25 mg/ml (right-hand-side image).
- **Figure 2:**: SEM image of chitosan-(hydroxypropyl-β-cyclodextrin) formulations. Formulation prepared from 25 mM of cyclodextrin and 2 mg/ml of tripolyphosphate.
- **Figure 3:**: Stability of nanoparticles of chitosan and cyclodextrin in HBSS at pH 6.4 at 37°C (mean±S.D., n=3). CS:chitosan; SBE-CD: sulfobutylether-cyclodextrin; CM-CD: carboxymethyl-cyclodextrin; TPP: sodium tripolyphosphate; HBSS: Hanks' balanced salt solution.
- **Figure 4:**: Stability of nanoparticles of chitosan and cyclodextrin in simulated intestinal fluid, at pH=6.8 at 37°C (mean±S.D., n=3). ( ) CS/CM-CD/TPP=4/5.5/0; (•) CS/CM-CD/TPP= 4/4.5/0.25. CS:chitosan; SBE-CD: sulfobutylether-β-cyclodextrin; CM-CD: carboxymethyl-β-cyclodextrin; TPP: sodium tripolyphosphate.
- **Figure 5:**: Stability of nanoparticles of chitosan and carboxymethyl-β-cyclodextrin in simulated intestinal fluid at pH 6.8 and 37°C (mean±S.D., n=3). ( ) CS/CM-CD/TPP=4/3/0.5; (•) CS/CM-CD/TPP= 4/1.5/0.75. CS:chitosan; CM-CD: carboxymethyl-β-cyclodextrin; TPP: sodium tripolyphosphate.
- **Figure 6:**: Release profile of the drugs triclosan and furosemide from chitosan-(hydroxypropyl cyclodextrin) formulations. Formulations: TRIC HPαCD (formulation of triclosan with hydroxypropyl-α-cyclodextrin), TRIC HPβCD (formulation of triclosan with hydroxypropyl-β-cyclodextrin), FUR HPαCD (formulation of furosemide with hydroxypropyl-α-cyclodextrin), FUR HPβCD (formulation of furosemide with hydroxypropyl-β-cyclodextrin) (Means ± Std. Dev., n=3).
- **Figure 7:**: Agarose gel of chitosan-sulfobutylcyclodextrin nanoparticles. Lines: (1) molecular weight marker, (2) DNA in solution, (3) nanoparticles without DNA, (4) nanoparticles with DNA, (5) nanoparticles with DNA degraded with chitosanase. Incubation time 30 minutes.
- **Figure 8:**: Fluorescence images of cells transfected with 1 µg of pGFP plasmid in nanoparticles of chitosan-sulfobutylcyclodextrin. Transfection levels achieved at 48 h.
- **Figure 9:**: Stability of nanoparticles of chitosan labelled with fluorescein-cyclodextrin in trehalose (5%). (◆) FI-CS/SBE-CD 4/4; ( ) FI-CS/CM-CD 4/6. FI-CS: fluorescein-labelled chitosan; SBE-CD: sulfobutylether-β-cyclodextrin; CM-CD: carboxymethyl-β-cyclodextrin; TPP: sodium tripolyphosphate.

### DETAILED DESCRIPTION OF THE INVENTION

The system of this invention comprises nanoparticles which are dispersed in an aqueous medium, with said nanoparticles having a structure that comprises chitosan and cyclodextrin, wherein a biologically active molecule may be incorporated. Said structure is joined by means of electrostatic interactions between both components, without any covalent bonds between them.

Optionally, the nanoparticles may comprise, in addition, an ionic crosslinking agent which allows for the crosslinking of chitosan by means of ionotropic gelation, thus favouring the spontaneous formation of nanoparticles.

The term "nanoparticle" is understood to be a structure formed by the electrostatic interaction between chitosan and cyclodextrin, where said structure may, in addition, be crosslinked when a polyanionic salt is added to the system which acts as a crosslinking agent. The resulting electrostatic interaction between the different nanoparticle components, and, optionally, the crosslinking of chitosan by the addition of a crosslinking agent, generates characteristic, independent, observable physical entities, the mean size whereof is less than 1 µm, i.e. a mean size of between 1 and 999 nm.

"Mean size" is understood to be the mean diameter of the population of nanoparticles comprised by chitosan and cyclodextrin, which move jointly in the aqueous medium. The mean size of these systems may be measured by standard procedures well-known by those skilled in the art, and which are described, for example, in the experimental part below.

The nanoparticles described in this invention are characterised in that they exhibit a mean particle size of less than 1 µm, preferably they have a mean size of between 1 and 999 nm, preferably between 10 and 800 nm. The mean size of the particles is primarily influenced by the proportion of chitosan with respect to cyclodextrin, the degree of deacetylation of chitosan and also by the particle formation conditions (concentration of chitosan, concentration of cyclodextrin, concentration of crosslinking agent, if any, and ratio between them).

On the other hand, the nanoparticles may exhibit an electric charge (measured by means of the Z potential, using CLK as a dilution medium), the magnitude whereof may vary from 0 mV up to + 60 mV, depending on the above-mentioned variables. The nanoparticles' positive charge may be of interest for favouring their interaction with biological surfaces and, particularly, with those mucous surfaces which are negatively charged. However, the neutral charge may be more suitable for the parenteral administration thereof.

The system comprising nanoparticles designed for the release of a biologically active molecule defined above has a chitosan content in the mixture greater than 40% by weight, preferably it is between at least 40% and 95.5% by weight. On the other hand, the cyclodextrin content in the mixture is less than 60% by weight, preferably it is between 0.5% and less than 60% by weight.

### Chitosan

Chitosan is a natural polymer derived from chitin (poly-N-acetyl-D-glucosamine), where a significant part of the N-acetyl groups have been eliminated by means of hydrolysis. The degree of deacetylation is preferably within a range of between 30 and 95%, more preferably between 50 and 95%, which indicates that between 5% and 50% of the amino groups are acetylated. Therefore, it exhibits an aminopolysaccharide structure and a cationic character. It comprises the repetition of monomer units of formula (I): 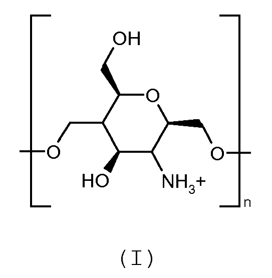 where n is an integer, and, in addition, m units wherein the amino group is acetylated. The sum of n+m represents the degree of polymerisation, i.e. the number of monomer units in the chitosan chain.

The chitosan used to produce the nanocapsules of this invention has a molecular weight of between 1 and 2,000 kDa, preferably between 5 and 500 kDa, more preferably between 5 and 200 kDa. Examples of commercial chitosans that may be used are UPG 113, UP CL 213 and UP CL113, which may be obtained from NovaMatrix, Drammen, Norway.

The number of monomer units which comprise the chitosan used to produce the nanoparticles is between 5 and 5000 monomers, preferably between 60 and 600 monomers.

As an alternative to chitosan, a derivative thereof may also be used, understanding such to be a chitosan wherein one or more hydroxyl groups and/or one or more amino groups have been modified, in order to increase the solubility of chitosan or increase the adhesive character thereof. These derivatives include, amongst others, acetylated, alkylated or sulfonated chitosans, thiolated derivatives, as described in Roberts, Chitin Chemistry, Macmillan, 1992, 166. Preferably, when a derivative is used, it is selected from O-alkyl ethers, O-acyl esters, trimethylchitosan, chitosans modified with polyethylene glycol, etc. Other possible derivatives are the salts, such as citrate, nitrate, lactate, phosphate, glutamate, etc. In any case, a person skilled in the art is able to identify the modifications that may be performed on the chitosan without affecting the stability and commercial viability of the final formulation.

### Cyclodextrin

Cyclodextrins structurally consist of 6, 7 or 8 units of D-glucopyranosyl joined by α(1-4) glycosidic bonds, which are called α, β or γ, respectively. The most stable three-dimensional configuration of these oligosaccharides is a toroid wherein the primary and secondary hydroxyl groups are oriented towards the solvent. In this configuration, the cavity formed within the toroid exhibits high hydrophobicity, which is responsible, jointly with Van der Waals forces and hydrogen bridges, for the formation of inclusion complexes between the cyclodextrins and the drugs.

A cyclodextrin derivative is understood to be a cyclodextrin or mixtures thereof wherein the hydrogen(s) of a part or all of the hydroxyl groups at positions 2-, 3- and 6- of glucose is(are) replaced by (an)other functional group(s), such as a dihydroxyalkyl group, a saccharide residue, a hydroxyalkyl group, a sulfonate group, a sulfoalkyl group, an alkyl group, an alkanoyl group, an acetyl group or a benzoyl group.

The cyclodextrin or its derivatives used in this invention may be commercially available or may be synthesised by a method known *per se.* Examples of cyclodextrin and its derivatives comprise natural cyclodextrins (alpha, beta or gamma), hydroxypropyl cyclodextrins, carboxymethylcyclodextrins, sulfobutylcyclodextrins, aminocyclodextrin, dimethylcyclodextrin, cyclodextrin phosphate, hydroxyethylcyclodextrin, acetyl-cyclodextrin, ethylcyclodextrins, trimethylcyclodextrins, carboxyethylcyclodextrin, glucosylcyclodextrin, 6-O-α-maltosylcyclodextrins, butyl-cyclodextrins, sulfated cyclodextrins, N,N-diethylaminoethylcyclodextrin, tert-butylsilylcyclodextrins, silyl[(6-O-tert-butyldimethyl)-2,3,-di-O-acetyl)-cyclodextrins, succinyl-(2-hydroxypropyl)-cyclodextrins, succinyl-cyclodextrins, sulfopropyl-cyclodextrins, polycyclodextrins. In a particular emodiment of this invention, the cyclodextrin is hydroxypropyl-α-cyclodextrin, hydroxypropyl-β-cyclodextrin, sulfobutylethyl-β-cyclodextrin or mixtures thereof. Any of them may be used in the systems of the invention.

The mean degree of substitution (DS) refers to the mean number of hydroxyls substituted per unit of cyclodextrin, whereas the degree of molar substitution (MS) refers to the number of hydroxyl groups per unit of anhydroglucose. In this invention, the cyclodextrins used exhibit a mean degree of substitution ranging from 4.2 to 7, although cyclodextrins with a DS beyond that range may also be applied.

The nanoparticle system of the invention is characterised in that it has been formed by spontaneous precipitation of the nanoparticles following mixing of a polycationic phase, which comprises chitosan and, optionally, cyclodextrin, with a polyanionic phase, which may be formed by a cyclodextrin or by a crosslinking agent or by a combination of both. It is significant that both phases are aqueous, thus avoiding or minimising the use of organic solvents in the preparation of the systems of the invention.

The crosslinking agent is an anionic salt which allows for the crosslinking of chitosan, favouring the spontaneous formation of nanoparticles. In this invention, the crosslinking agent is a polyphosphate salt, with the use of sodium tripolyphosphate (TPP) being preferred.

When the cyclodextrin is anionic, it may form the anionic phase by itself, and the presence of TPP is then not necessary, since the nanoparticles are formed by electrostatic interaction between the negatively charged cyclodextrins and the positively charged chitosan. However, the addition of TPP, jointly with anionic cyclodextrin, may in some cases change the crosslinking density and favour the nanoparticles' stability. On the other hand, in the case of cyclodextrins without an anionic charge (without any charge or with a positive charge), it is necessary to incorporate TPP in the polyanionic phase in order to crosslink the chitosan and allow for the formation of the nanoparticles.

The nanoparticles of chitosan-cyclodextrin are systems with a high capacity to associate with bioactive molecules. This association capacity depends on the type of molecule incorporated, as well as the specified formulation parameters. In this invention, this type of nanoparticles is particularly aimed at associating hydrophobic active molecules and low-permeability active molecules, whether hydrophobic or hydrophilic. Therefore, a second aspect of this invention is a nanoparticle system such as the one defined above which, in addition, comprises a biologically active molecule.

The term "biologically active molecule" refers to any substance which is used in the treatment, curing, prevention or diagnosis of a disease or which is used to improve the physical and mental well-being of humans and animals. These biologically active molecules may include from low-molecular-weight drugs to molecules of the polysaccharide, protein, peptide, and lipid types, and nucleic acid-based molecules and combinations thereof.

In a particular embodiment, the biologically active molecules are drugs pertaining to class II (non-permeable water-soluble), class III (permeable hydrophobic) and, preferably, class IV (non-permeable hydrophobic), according to the FDA definition.

The biologically active molecules which may be used with the system of the invention include, amongst others, the following class II molecules: Danazol; Ketoconazole; mefenamic acid; Nisoldipine; Nifedipine; Nicardipine; Felodipine, Atovaquone, Griseofulvin, Troglitazone, Glybenclamide, Carbamazepine; class III molecules: Acyclovir; Neomycin B; Captopril; Enalaprilate; Alendronate, Atenolol, Cimetidine, Ranitidine; class IV molecules: Chlorothiazide; Furosemide; Tobramycin, Cefuroxime, Itraconazole, Cyclosporin.

In a preferred embodiment, the biologically active molecule is triclosan. In another preferred embodiment, the biologically active molecule is furosemide.

In another particular embodiment, the biologically active molecules are macromolecules of the peptide, polysaccharide, or protein type or nucleic acid-based (oligonucleotides, DNA, siRNA).

In a preferred embodiment, the biologically active molecule is insulin. In another preferred embodiment, the biologically active molecule is heparin. In another preferred embodiment, the biologically active molecule is DNA.

Another aspect of the present invention is a vaccine which comprises the nanoparticle system defined above and an antigen. The administration of an antigen by the system composed of the nanoparticles makes it possible to achieve an immune response. The vaccine may comprise a protein, a polysaccharide, or it may be a DNA vaccine. Strictly speaking, a DNA vaccine is a DNA molecule which encodes the expression of an antigen which shall give rise to an immune response.

The association of the biologically active molecule may take place by means of combined processes which comprise non-covalent interactions between the active molecule and the polymer or the association of the active molecule with a cyclodextrin, forming an inclusion complex, and the non-covalent interaction of this complex with the polymer matrix.

In order to adequately incorporate the biologically active molecule to the nanoparticles of chitosan, using the active-molecule-cyclodextrin complexation approach, it is necessary to first dissolve a reasonable quantity of active molecule due to its complexation with cyclodextrin and, subsequently, encapsulate a sufficient quantity of the complex in the nanoparticle structure.

Another object of this invention is a pharmaceutical composition which comprises the nanoparticle system defined above and a biologically active molecule capable of preventing, palliating or curing diseases.

Examples of pharmaceutical compositions include any liquid (suspension of nanoparticles) or solid (lyophilised or atomised nanoparticles, forming a powder which may be used to prepare granulates, tablets or capsules) composition for administration by oral, buccal or sublingual route, or in liquid or semi-solid form for administration by transdermal, ocular, nasal, vaginal or parenteral route. In the case of non-parenteral routes, contact of the nanoparticles with the skin or mucous membranes may be improved by endowing the particles with a significant positive charge, which shall favour their interaction with the above-mentioned negatively charged surfaces. In the case of parenteral routes, more specifically, intravenous administration, these systems offer the possibility to modulate the *in vivo* distribution of the drugs or molecules which may be associated therewith.

In a preferable aspect, the pharmaceutical composition is administered by transmucosal route. The positive charge of the chitosan-cyclodextrin mixture provides a better absorption of the drugs on the mucous surface through their interaction with the mucous membrane and the surfaces of the negatively charged epithelial cells.

The proportion of active ingredient incorporated in the nanoparticles may be up to 40% by weight with respect to the total weight of the system. However, the suitable proportion will depend in each case on the active ingredient which is to be incorporated, the indication it is designed for and the release efficiency.

The nanoparticle systems of this invention may also incorporate cosmetically active molecules which do not exhibit a therapeutic effect, but lead to cosmetic compositions. These cosmetic compositions include any liquid composition (suspension of nanoparticles) or emulsion for topical administration. Amongst the cosmetically active molecules which may be incorporated to the nanoparticles, one may cite anti-acne agents, antifungal agents, antioxidant agents, deodorants, antiperspirants, anti-dandruff agents, skin-whiteners, tanning lotions, UV-light absorbers, enzymes, cosmetic biocides, amongst others.

Another aspect of this invention relates to a method for the preparation of nanoparticles of chitosan-cyclodextrin such as those defined above, which comprises:
a) preparation of a solution of chitosan or a derivative thereof in an aqueous medium or in a mixture of water with a polar solvent;
b) preparation of a solution of cyclodextrin or a derivative thereof in an aqueous medium or in a mixture of water with a polar solvent and, optionally, a crosslinking agent; and
c) mixing, under stirring, of the solutions of steps a) and b) such that the nanoparticles of chitosan-cyclodextrin are spontaneously produced,
or, optionally:
a. preparation of a solution of chitosan or a derivative thereof and a cyclodextrin or a derivative thereof in an aqueous medium or in a mixture of water with a polar solvent;
b. preparation of a solution of the crosslinking agent in an aqueous medium or in a mixture of water with a polar solvent;
c. mixing, under stirring, of the solutions of steps a) and b) such that the nanoparticles of chitosan-cyclodextrin are spontaneously produced.

Non-toxic solvents may be used as polar solvents, including, amongst others, acetonitrile, alcohols and acetone. Similarly, the aqueous medium used may contain different types of salts.

In a variant of the method, the resulting chitosan/cyclodextrin/crosslinking agent mass ratio is between 4/4/1 and 4/80/1. However, the use of higher ratios of chitosan with respect to cyclodextrin or to the crosslinking agent is also possible, depending on the type of cyclodextrin used. Thus, for neutral cyclodextrins (such as HPβCD), the presence of cyclodextrin does not seem to affect the process of formation of the nanoparticles.

The biologically active molecule may be directly incorporated to the solutions of steps a) or b), such that the nanoparticles of chitosan-cyclodextrin containing the biologically active molecule are spontaneously produced. However, in a variant of the method, the molecule may be dissolved in a previous step in an aqueous phase or in a mixture of an aqueous phase and a polar solvent and incorporated to steps a) or b) prior to preparing the particles (step c)). However, for low-solubility drugs, higher concentrations are achieved if the active molecule is dissolved in the same step with cyclodextrin.

The method of preparation of the nanoparticles of chitosan-cyclodextrin may also comprise an additional step, wherein said nanoparticles are lyophilised. From a pharmaceutical standpoint, it is important to have nanoparticles in lyophilised form available, since this improves their stability during storage and reduces the volume of product to be handled. The nanoparticles of chitosan-cyclodextrin may be lyophilised in the presence of a cryoprotector, such as glucose, saccharose or trehalose, at a concentration ranging between 1 and 5% by weight. In fact, the nanoparticles of the invention have the additional advantage that the particle size before and after lyophilisation is not significantly affected. That is, the nanoparticles have the advantage of being lyophilised and resuspended without suffering any alteration in the physical characteristics thereof.

The system of the present invention has proven to be a highly efficient carrier in interacting with epithelial cells and promoting the transfection of a polynucleotide in a cell. The nanoparticles comprised in the system may incorporate genetic material in the cell, such as a nucleic acid-based molecule, an oligonucleotide, siRNA or a polynucleotide, preferably a DNA plasmid that encodes a protein of interest, which allows the system to be potentially suitable for use in gene therapy. In a particular embodiment, the DNA plasmid is pEGFP.

*In vitro* studies have made it possible to observe a very efficient release of the DNA plasmid, achieving significant levels of cell transfection. Consequently, another aspect of the invention relates to the use of the nanoparticle system of the invention in the preparation of a gene therapy drug. In a particular aspect, it comprises a polynucleotide comprising a gene capable of functionally expressing itself in the cells of the patient to be treated.

In this regard, some examples of diseases which may be treated using the system of the invention are macular degeneration with anti-VEGF antisense drugs, bullous epidermolysis and cystic fibrosis. It may also be used in the healing of wounds with transitory transformation schemes.

Finally, due to their high transfection capacity, the system and the compositions of the invention, which contain synthetic or natural polynucleotides, may be used in the transfection of target cells, preferably neoplastic or "normal" mammal cells, as well as stem cells or cell lines. Moreover, it is a useful tool for the genetic manipulation of cells. In this regard, the invention also relates to the use of the system of the invention for the genetic manipulation of cells. Preferably, it is used for the release of nucleic acids *in vitro* or *ex vivo.* Such release is aimed at target cells, which comprise: eukaryotic cells, such as mammal cells, cell lines, and may lead to *in vitro* or *ex vivo* cell transfection or transformation. Therefore, the invention is also related to a kit designed for the transfection of eukaryotic cells, which comprises the system of the invention and adequate diluents and/or buffers for cell washing.

Below we describe some illustrative examples of the invention; however, they should not be considered as imposing limitations thereon.

### EXAMPLES

The physicochemical properties of the formulations with different compositions and different polymer ratios have been characterised using photon correlation spectroscopy (PCS) and laser-Doppler anemometry techniques. The nanoparticles' morphology was studied by means of transmission electronic microscopy (TEM) and scanning electronic microscopy (SEM). The composition of the nanoparticles prepared was studied using elementary analysis techniques. This study proved the presence of chitosan-cyclodextrin mixtures in the nanoparticle matrices.

### Example 1.

### Evaluation of the characteristics of nanoparticles of chitosan-cyclodextrin as a function of the type of chitosan and the concentration of TTP.

Fixed-concentration (6.29mM) solutions (3 ml) of hydroxypropyl-β-cyclodextrin (HPβCD) were prepared with different chitosans (CS) (0.2% w/w). These solutions were incubated for 24 h under magnetic stirring and, subsequently, were filtered with a 0.45-µm filter and crosslinked by the addition of different volumes of tripolyphosphate at concentrations of 1.25 mg/ml or 2 mg/ml, such that a chitosan/tripolyphosphate mass ratio of 4:1 was always maintained. The nanoparticles were isolated by centrifugation at 16000xg and resuspended in water. The size of the nanoparticles was determined by means of photon correlation spectroscopy (PCS). The results related to the mean size and the polydispersion index of the nanoparticles as a function of the molecular weight of the chitosan used and of the concentration of the tripolyphosphate used as a crosslinking agent are shown in table 1.

**Table 1: Effect of the molecular size of chitosan (CS Mw), the presence of HPβCD and the concentration of the crosslinking agent tripolyphosphate (TPP) on the mean size and the polydispersion of the nanoparticles (Mean ±std. dev., n=3).**

| CS Mw (KDa) | HPβCD concentration (mM) | TPP concentration (mg/ml) | size (nm) | polydispersion index (P.I.) |
|---|---|---|---|---|
| 110 | 0 | 1.25 | 484 ± 32 | 0.3 |
| 110 | 6.29 | 1.25 | 454 ± 19 | 0.3 |
| 110 | 0 | 2.0 | 578 ± 1 | 0.3 |
| 110 | 6.29 | 2.0 | 590 ± 1 | 0.2 |
| 272 | 0 | 2.0 | 887 ± 5 | 0.5 |
| 272 | 6.29 | 2.0 | 808 ± 0 | 0.5 |

### Example 2.

### Evaluation of the characteristics of nanoparticles of chitosan-cyclodextrin as a function of the type and the concentration of cyclodextrin (concentration of TTP = 2 mg/ml).

0.2% (w/w) solutions (3 ml) of chitosan, specifically chitosan hydrochloride (Protasan C1110), were prepared with different quantities of hydroxypropyl cyclodextrin (α- or β-) (0 to 25 mM). The solutions were incubated for 24 h under magnetic stirring and, subsequently, were filtered through a 0.45-µm pore size and crosslinked by the addition of 0.75 ml of tripolyphosphate at concentrations of 2 mg/ml. The nanoparticles were isolated by centrifugation at 16000xg and resuspended in water. The size of the resulting particles and the polydispersion thereof were characterised by means of photon correlation spectroscopy (PCS), the zeta potential by means of laser-Doppler anemometry and the production yield by weighing the dry residue of a sample of isolated nanoparticles. The results are shown in table 2.

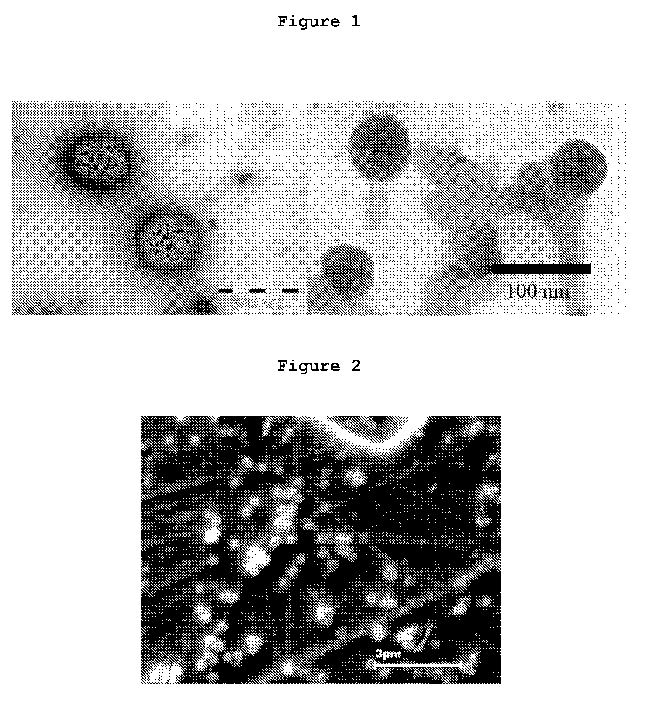

Figure 1 (left-hand-side image) and Figure 2 show the morphology of particles prepared from 25 mM of HPβCD analysed by means of TEM and SEM, respectively, confirming the formation of spherical nanoparticles.

**Table 2: Effect of the type and the concentration of hydroxypropyl cyclodextrin on the characteristics of chitosan-cyclodextrin nanoparticles (size, polydispersion, zeta potential and production yield) (Means ± std. dev., n=3).**

| HPCD concentration (mM) | type HPCD | size (nm) | polydispersion index (P.I.) | zeta potential (mV) |
|---|---|---|---|---|
| 0 | ^{~~~} | 686 ± 1 | 0.5 | +33.8 ± 3.4 |
| 3.14 | α | 625 ± 4 | 0.6 | +34.7 ± 2.3 |
| | β | 590 ± 1 | 0.3 | +35.3 ± 3.8 |
| 6.29 | α | 645 ± 7 | 0.6 | +33.8 ± 0.5 |
| | β | 624 ± 0 | 0.3 | +36.2 ± 0.5 |
| 25 | α | 690 ± 3 | 0.4 | +35.3 ± 3.8 |
| | β | 670 ± 4 | 0.6 | +33.1 ± 3.3 |

### Example 3.

### Evaluation of the characteristics of nanoparticles of chitosan-cyclodextrin as a function of the type and the concentration of cyclodextrin (concentration of TPP = 1.25 mg/ml).

0.2% (w/w) solutions (3 ml) of chitosan (Protasan C1110) were prepared with different quantities of hydroxypropyl cyclodextrin (α- or β-) (0 to 25 mM). The solutions were incubated for 24 h under magnetic stirring and, subsequently, the solutions were filtered through a 0.45-µm pore size and crosslinked by the addition of 1.2 ml of tripolyphosphate at concentrations of 1.25 mg/ml. The nanoparticles were isolated by centrifugation at 16000xg and resuspended in water. The size of the resulting particles and the polydispersion thereof were characterised by means of photon correlation spectroscopy (PCS), the zeta potential by means of laser-Doppler anemometry and the production yield by weighing the dry residue of a sample of isolated nanoparticles. The results are shown in table 3.
Figure 1 (right-hand-side image) shows the morphology of particles prepared from 25 mM of HPβCD analysed by means of TEM.

**Table 3: Effect of the type and the concentration of hydroxypropyl cyclodextrin on the characteristics of chitosan-cyclodextrin nanoparticles (size, polydispersion, zeta potential and production yield) (Means ± Std. Dev., n=3).**

| HPCD concentration (mM) | type HPCD | size (nm) | polydispersion index (P.I.) | zeta potential (mV) | production yield (%) |
|---|---|---|---|---|---|
| 0 | --- | 484 ± 32 | 0.3 | +37.6 ± 0.9 | 42 ± 7 |
| 3.14 | α | 410 ± 29 | 0.2 | +36.9 ± 0.6 | 45 ± 6 |
| | β | 456 ± 37 | 0.3 | +34.2 ± 1.0 | 51 ± 6 |
| 6.29 | α | 398 ± 14 | 0.2 | +35.9 ± 3.8 | 48 ± 7 |
| | β | 454 ± 19 | 0.3 | +34.8 ± 3.2 | 54 ± 4 |
| 25 | α | 361 ± 18 | 0.2 | +35.8 ± 1.7 | 65 ± 9 |
| | β | 443 ± 27 | 0.5 | +29.8 ± 2.9 | 74 ± 3 |

The results obtained in examples 2 and 3 show that the inclusion of cyclodextrins affects the size of the resulting nanoparticles but without excessively changing the value thereof. Regarding the Z potential, the nanoparticles prepared in the presence of cyclodextrins present very similar values. This data allow us to deduce that the cyclodextrins do not interfere with the process of formation of the nanoparticles and that they are not necessarily associated therewith.
On the other hand, the production yield significantly increases when the concentration of cyclodextrin increases.

### Example 4.

### Stability of nanoparticles of chitosan and cyclodextrin in cell cultures.

Nanoparticles of chitosan were prepared with two types of cyclodextrin by mixing an aqueous solution of sulfobutylether-β-cyclodextrin (SBE-CD) or of carboxymethyl-β-cyclodextrin (CM-CD) with an aqueous solution of chitosan (CS) under magnetic stirring in the presence of the crosslinking agent TPP in such a way that the ratio between the different components is:

| | |
|---|---|
| CS/SBE-CD/TPP: | (4/3/0.25) |
| CS/CM-CD)/TPP: | (4/4/0.25) |

Subsequently, the nanoparticles were isolated by centrifugation and, subsequently, they were incubated in a Hanks' salt solution (HBSS) at 37°C. This buffered solution (which contains inorganic salts and glucose) is probably the most widely used in experiments with cell cultures, since it makes it possible to maintain the cells at a physiological pH and osmotic pressure, thus preserving them in a viable state for short periods of time without promoting their growth. The stability studies were conducted by measuring the change in size of the nanoparticles.

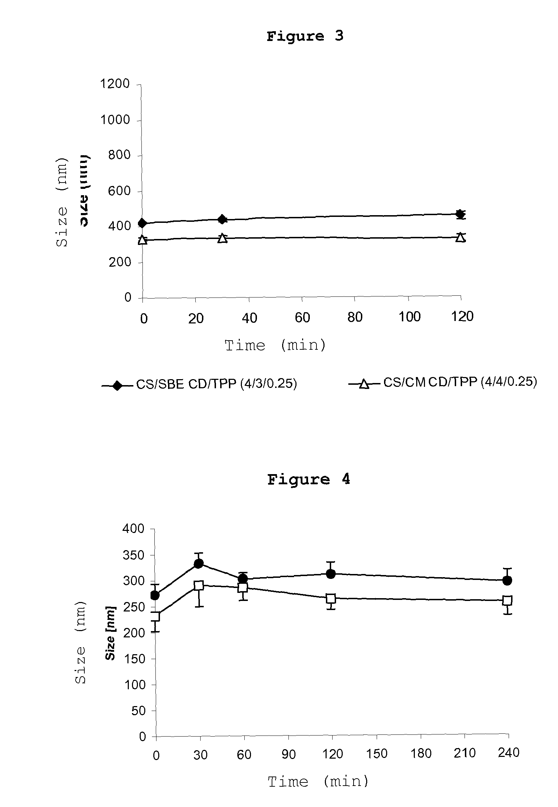

As shown in figure 3, the nanoparticles of chitosan and cyclodextrin were stable under the experimental conditions.

### Example 5.

### Stability of nanoparticles of chitosan and cyclodextrin in simulated intestinal fluid.

Nanoparticles of chitosan and carboxymethyl-β-CD were prepared as described in example 4 by means of ionic gelation, in the presence and in the absence of TPP. The stability of these nanoparticles was evaluated in a simulated intestinal fluid at pH=6.6 and 37°C. This medium reproduces the conditions of the small intestine, but may also reflect the stability of the nanoparticles on the nasal mucous membrane. The nanoparticles proved to be stable for over 4 hours, as shown in figure 4, for which reason they appear to be suitable systems for different administration routes.

### Example 6.

### Evaluation of the encapsulation capacity of insulin in nanoparticles of chitosan and cyclodextrin.

Nanoparticles of chitosan and carboxymethyl-β-CD were prepared as described in example 4 or 5 using different concentrations of cyclodextrin and of TPP and, in some cases, incorporating a 0.24% concentration of insulin to the initial aqueous solutions. Subsequently, the nanoparticles were isolated by centrifugation. Table 4 shows the physicochemical characteristics of the nanoparticles, loaded or not loaded with insulin.

**Table 4: Physicochemical characteristics of the nanoparticles of CS/CM-CD/TPP, loaded or not loaded with insulin. (*) nanoparticles loaded with insulin**

| CS/CM-CD/TPP | size (nm) | polydispersion index | zeta potential (mV) |
|---|---|---|---|
| 4/3/0 | 200±13 | 0.11-0.16 | +2.0±1.4 |
| 4/4/0 | 238±16 | 0.08-0.10 | +27.0±2.4 |
| 4/3.5/0 (*) | 482±33 | 0.04-0.19 | +29.6±0.8 |
| 4/2/0.5 | 299±25 | 0.36-0.46 | +32.0±0.3 |
| 4/3/0.25 | 264±18 | 0.23-0.37 | +27.0±0.6 |
| 4/4/0.25 (*) | 436±34 | 0.10-0.23 | +25.9±1.8 |
| 4/3/0.5 (*) | 555±119 | 0.02-0.52 | +31.4±1.4 |
| 4/2/0.75 (*) | 631±153 | 0.29-0.41 | +31.2±1.5 |
| 4/1.5/0.75 (*) | 613±124 | 0.11-0.58 | 31.0±1.5 |
| 4/0/1 (*) | 454±120 | 0.22-0.31 | 37.1±1.3 |

As can be observed, the size of the loaded nanoparticles ranges between 430 and 635 nm, said size being up to two times greater than that of the nanoparticles not loaded with insulin.

On the other hand, table 5 shows the loading capacity of insulin in nanoparticles. One may observe that insulin may be very efficiently incorporated to the nanoparticles, exhibiting association efficiencies of over 85%.

**Table 5: Efficiency in the encapsulation of insulin in nanoparticles of CS/CM-CD/TPP (concentration of insulin 0.24%).**

| CS/CM-CD/TPP | loading capacity (%) | association efficiency (%) | yield (%) |
|---|---|---|---|
| 4/3.5/0 | 68.4±0.5 | 85.5±0.4 | 22.6 |
| 4/4/0.25 | 46.7±0.8 | 88.6±0.8 | 33.0 |
| 4/3/0.5 | 38.5±0.4 | 92.6±0.6 | 41.7 |
| 4/2/0.75 | 33.1±0.1 | 94.7±0.2 | 57.3 |
| 4/1.5/0.75 | 38.7±0.5 | 93.3±0.7 | 50.9 |
| 4/0/1 | 34.7±0.3 | 91.4±0.4 | 69.3 |

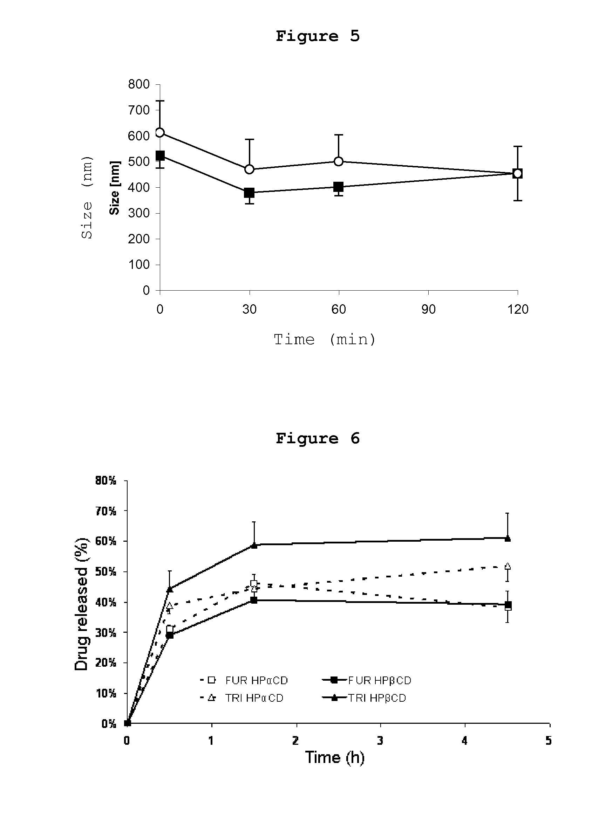

Furthermore, the stability of the nanoparticles of CS/CM-CD/TPP loaded with insulin was evaluated in simulated intestinal fluid at pH 6.8 and 37°C, as described in example 5. The size of the nanoparticles did not increase with respect to the initial size within the first two hours (Figure 5).

### Example 7.

### Evaluation of the solubility of triclosan, the encapsulation efficiency and the load thereof in nanoparticles as a function of the type and the concentration of cyclodextrin.

Nanoparticles of chitosan-cyclodextrin were obtained according to the method described in example 2, but adding a sufficient quantity of the drug triclosan to the initial solutions in order to oversaturate the solution. The drug that was not dissolved by the cyclodextrin-polymer mixture was discarded during the filtration process (through 0.45 µm) which was conducted prior to crosslinking the polymer by means of ionotropic crosslinking (see example 2). Table 6 shows the solubility achieved for triclosan in the initial solutions used for the formation of the particles, the encapsulation efficiency of triclosan in the nanoparticles and the load of triclosan achieved in these nanoparticles. The triclosan was measured by means of a spectrophotometric method (λ=280 nm).

The encapsulation efficiency (EE) refers to the percentage of drug which is trapped in the chitosan-cyclodextrin system with respect to the quantity of drug added in the process of preparation of the nanoparticles. The drug load is indirectly determined from a calculation of the non-encapsulated drug which remains dissolved in the nanoparticles' suspension medium. The difference between this value and the theoretical drug content is taken to be the quantity of drug loaded in the nanoparticles. The drug load percentage which appears in the table is the percentage with respect to the quantity of encapsulated drug in 100 mg of nanoparticle.

**Table 6: Effect of the type and the concentration of the cyclodextrin used in the solubilisation of triclosan, the resulting encapsulation efficiency (EE) and the load thereof in the final nanoparticles. (Mean ± Std. Dev., n=3).**

| type HPCD | HPCD concentration (mM) | solubility of triclosan (mg/l) | triclosan EE (%) | triclosan load (%) |
|---|---|---|---|---|
| --- | 0 | 68 ± 17 | 12.5 ± 8 | 0.8 ± 0.3 |
| β | 3.14 | 211 ± 24 | 5.2 ± 7 | 1.1 ± 0.2 |
| β | 6.29 | 588 ± 18 | 4.8 ± 3 | 2.2 ± 0.1 |
| β | 25 | 1120 ± 13 | 5.5 ± 5 | 3.1 ± 0.1 |
| α | 25 | 870 ± 59 | 4.6 ± 4 | 2.0 ± 0.1 |

### Example 8.

### Evaluation of the solubility of furosemide, the encapsulation efficiency and the load thereof in nanoparticles as a function of the type and the concentration of cyclodextrin.

Nanoparticles of chitosan-cyclodextrin were prepared according to the method described in example 3, but adding a sufficient quantity of the drug furosemide to the initial solutions in order to oversaturate the solution. The drug that was not dissolved by the cyclodextrin-polymer mixture was discarded during the filtration process (through 0.45 µm) which was conducted prior to crosslinking the polymer (see example 3). Table 7 shows the solubility achieved for furosemide in the initial solutions used for the formation of the particles, the encapsulation efficiency of furosemide in the nanoparticles and the load of furosemide achieved in these nanoparticles. The furosemide was measured by means of a spectrophotometric method (λ=230 nm). In order to determine the quantity of encapsulated furosemide, the quantity of triclosan in the particles' supernatant following the isolation thereof (non-associated quantity) was determined and the difference was calculated.

**Table 7: The effect of the type and the concentration of the cyclodextrin used in the solubilisation of furosemide, the resulting encapsulation efficiency (EE) and the load thereof in the final nanoparticles. (Mean ± Std. Dev., n=3).**

| type HPCD | HPCD concentration (mM) | solubility furosemide (mg/l) | furosemide EE (%) | load of furosemide (%) |
|---|---|---|---|---|
| --- | 0 | 7.8 ± 1.3 | 22.3 ± 1.4 | 0.23 ± 0.07 |
| β | 3.14 | 42.3 ± 2.4 | 17.1 ± 3.0 | 0.89 ± 0.04 |
| β | 6.29 | 95.4 ± 10.1 | 12.1 ± 1.3 | 1.43 ± 0.24 |
| β | 25 | 387.3 ± 10.3 | 7.2 ± 3.1 | 2.39 ± 0.72 |
| α | 25 | 253.5 ± 9.8 | 8.8 ± 2.7 | 1.92 ± 0.55 |

### Example 9.

### Release of the drug triclosan or furosemide from the nanoparticles of chitosan-cyclodextrin.

Nanoparticles of chitosan-cyclodextrin were prepared with triclosan and furosemide. In order to prepare the formulation with triclosan, the process described in example 7 was followed (formulations with 25 mM of HPCD α or β) and, for the formulation of furosemide, the method described in example 8 was followed (formulations with 25 mM of HPCD α or β). The nanoparticles were isolated and resuspended in an acetate buffer (pH 6.0, low ionic strength). The nanoparticles were incubated in this medium under horizontal stirring (100 rpm) at 37°C. At various times (0.5, 1.5 and 4.5 h), samples were taken from the incubation mediums, the drug was isolated in solution (centrifugation at 200000xg for 30 min) and assessed by spectrophotometric means, as described in examples 7 and 8. The drug release profile of the prepared formulations is shown in figure 6.

### Example 10.

### Evaluation of the solubility of triclosan, of the encapsulation efficiency and the load thereof in nanoparticles as a function of the type and the concentration of cyclodextrin.

In a mixture of 80% water and 20% ethanol, solutions (3 ml) of chitosan (Protasan C1110, 0.2%), HPβCD (0, 1.28 and 2.56 mM) and triclosan were prepared in a sufficient quantity to oversaturate the solution. These solutions were incubated for 24 h under magnetic stirring and, subsequently, filtered through a 0.45-µm filter and crosslinked by the addition of 1.2 ml of tripolyphosphate dissolved in a mixture of 80% water and 20% ethanol at a concentration of 1.25 mg/ml. The nanoparticles were isolated by centrifugation at 16000xg and resuspended in water. The size of the resulting particles and the polydispersion thereof were characterised by means of photon correlation spectroscopy (PCS). The quantity of encapsulated drug was determined by the degradation of an aliquot of the resuspended nanoparticles with the enzyme chitosanase (Chitosanase-RD, Pias Co, Japan), and the assessment was performed by means of spectrophotometry (λ=280 nm). The results are shown in table 8.

**Table 8: The effect of the concentration of the cyclodextrin used in the solubilisation of triclosan in the phase used for preparation of the nanoparticles, the resulting encapsulation efficiency of the nanoparticles (EE) and the final load in the nanoparticles. (Mean ± Std. Dev., n=3).**

| HPβCD concentration (mM) | size (nm) | dissolved triclosan (mg/l) | triclosan EE (%) | load of triclosan (%) |
|---|---|---|---|---|
| 0 | 499 ± 33 | 719 ± 79 | 22.3 ± 1.4 | 2.2 ± 0.9 |
| 1.28 | 568 ± 25 | 2536 ± 283 | 37.7 ± 7.3 | 7.4 ± 1.3 |
| 2.56 | 517 ± 14 | 3521 ± 213 | 33.5 ± 2.7 | 8.7 ± 0.2 |

### Example 11.

### Evaluation of the size and the polydispersion of nanoparticles of chitosan-cyclodextrin with or without plasmid DNA as a function of the type of cyclodextrin.

The following solutions were prepared: (A) methyl-β-cyclodextrin (Me-β-CD) (7.4 mM), tripolyphosphate (1.25 mg/ml) and a plasmid encoding the green fluorescent protein (pGFP) (0.5 mg/ml); and (B) sulfobutyl-β-cyclodextrin (0.18 mM) (SB-β-CD) and a plasmid encoding the green fluorescent protein (pGFP) (0.5 mg/ml). Both solutions were incubated for 1 h under stirring. A 0.24-ml volume of solutions A or B was added to 1.2 ml of 0.1% (w/w) chitosan under magnetic stirring, forming nanoparticles. The size and the polydispersion of the resulting particles were characterised by means of photon correlation spectroscopy (PCS). The results are shown in table 9.

**Table 9: The effect of the type of cyclodextrin on the size and the polydispersion of nanoparticles of chitosan-cyclodextrin with or without plasmid DNA. (Mean ± Std. Dev., n=3). *Expressed as a range of values.**

| type cyclodextrin | DNA concentration (mg/ml) | size of particle (nm) | polydispersion index (P.I.)* |
|---|---|---|---|
| SB-β-CD | 0 | 170.8 ± 24 | 0.1- 0.2 |
| SB-β-CD | 0.5 | 157 ± 32 | 0.1- 0.2 |
| Me-β-CD | 0 | 232 ± 15 | 0.2- 0.3 |
| Me-β-CD | 0.5 | 182.8 ± 40 | 0.1- 0.2 |

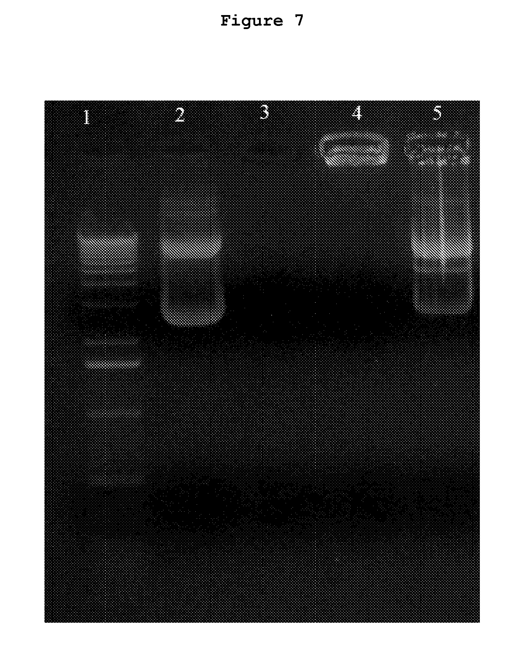

The formulation of nanoparticles of chitosan-sulfobutylcyclodextrin with DNA was subject to electrophoresis in an agarose gel prior to the isolation thereof. The controls included a plasmid in solution, the formulation without a plasmid and the formulation with a plasmid degraded with chitosanase (Chitosanase-RD, Pias Co, Japan). The results are shown in Figure 7.

### Example 12.

### In vitro study of the efficiency of transfection of cell cultures.

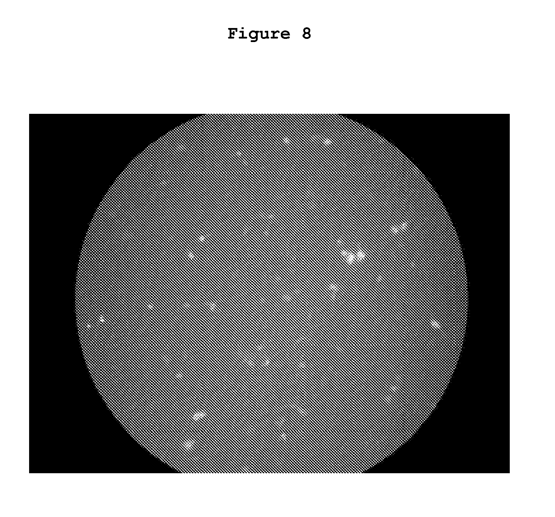

A formulation of nanoparticles such as the one described in example 11 was prepared. The formulation was isolated by centrifugation (16000xg, 30 min) and resuspended in a low ionic strength pH 6.0 buffer. A quantity of formulation containing 1 or 2 µg of DNA was incubated with cell cultures. The results for the cell transfection achieved are shown in figure 8. The fluorescence image shows the cell colonies which express the green fluorescent protein as a consequence of transfection by the nanoparticle-pGFP system. The plasmid without a carrier did not exhibit a capacity to transfect the cells, i.e. no fluorescent cell colonies were observed.

### Example 13.

### Effect of the type of cyclodextrin and the mass ratio between chitosan, cyclodextrin and tripolyphosphate in the final composition of the nanoparticle systems.

Nanoparticles of chitosan-HPβCD and chitosan-SBβCD were prepared as in examples 3 and 11, respectively. Different quantities of cyclodextrins in the initial solutions used to prepare the nanoparticles were used. The real composition of the nanoparticles (% chitosan, % cyclodextrin, % counterions) following the isolation thereof was determined by elementary analysis techniques (taking into consideration the Nitrogen-Carbon or Nitrogen-Sulphur ratios). The degree of humidity of the samples was determined by means of thermogravimetric analysis.

**Table 10: The effect of the type of cyclodextrin and of the mass ratio between chitosan (CS), cyclodextrin (CD) and tripolyphosphate (TPP) in the final composition of the prepared systems (% of dry weight). (Mean ± Std. Dev., n=3). *Approximately zero.**

| type cyclodextrin | initial CS/CD/TPP mass ratio | % CS | % cyclodextrin | % counterions (TPP, Na, Cl) |
|---|---|---|---|---|
| HPβCD | 4/2/1 | 70 ± 1 | 2.8 ± 1.1 | 27 ± 0.5 |
| HPβCD | 4/4/1 | 72 ± 0.4 | 4.2 ± 0.5 | 24 ± 0.5 |
| HPβCD | 4/8/1 | 70 ± 2 | 10.1 ± 2.5 | 20 ± 0.8 |
| SB-β-CD | 4/2/0.5 | 58 ± 2 | 31.7 ± 1.0 | 10.5 ± 1.9 |
| SB-β-CD | 4/3/0.5 | 46 ± 3 | 37.1 ± 7.9 | 16.7 ± 9 |
| SB-β-CD | 4/4/0 | 41 ± 0.2 | 58.6 ± 0.7 | ---* |

### Example 14.

### Study of the transport of nanoparticles of chitosan-cyclodextrin through the nasal mucous membrane of rats.

In order to evaluate the potential of the nanoparticle systems of the invention as carriers designed for the administration of drugs, the ability of said nanoparticles to cross the nasal epithelium was examined. This study was conducted with two specific formulations of chitosan/sulfobutylethyl-β-cyclodextrin (CS/SBE-β-CD) and chitosan/carboxymethyl-β-cyclodextrin (CS/CM-β-CD), using different proportions of the components. The chitosan was previously labelled with fluorescein (FI-CS). The labelling process was performed by reaction of carbodiimide with EDAC, which allowed for covalent bonding of the fluorescent label to the chitosan molecules, as described in Pharm. Res., 2004, 21, 803-10. Table 11 shows the physicochemical characteristics of the evaluated nanoparticles labelled with fluorescein.

**Table 11. Physicochemical characteristics of the evaluated nanoparticles labelled with fluorescein.**

| type of cyclodextrin, CD | FI-CS/CD | size (nm) | polydispersion index | yield (%) |
|---|---|---|---|---|
| SBE-β-CD | 4/3 | 219 | 0.09 | 22 |
| | 4/4 | 239 | 0.07 | 43 |
| CM-β-CD | 4/5 | 311 | 0.22 | 13 |
| | 4/6 | 309 | 0.41 | 24 |

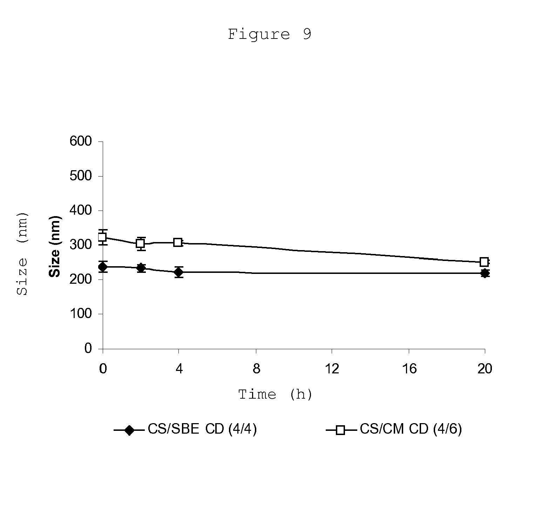

A suspension of these nanoparticles (the stability whereof was previously evaluated in a transport medium of 5% w/w trehalose, figure 9) was administered by intranasal route to fully awake rats. After a pre-set time had elapsed (specifically, 10 min following administration), the rats were put to sleep by cervical dislocation and the nasal mucous membrane was fixated with paraformaldehyde, excised and subsequently observed with a confocal microscope (CLSM, Zeiss 501, Jena, Germany) at 488 nm. The images observed showed that these nanoparticles exhibited a significant interaction with the nasal mucous membrane.

## Claims

1. A system which comprises nanoparticles for the release of biologically active molecules, where the nanoparticles comprise a) at least 40% by weight of chitosan or a derivative thereof and b) less than 60% by weight of a cyclodextrin or a derivative thereof, where both components a) and b) are mixed, without any covalent bond between them.

2. System, according to claim 1, where the nanoparticles further comprise an anionic salt capable of ionically crosslinking the chitosan in the form of nanometric structures.

3. System, according to claims 1 and 2, where the proportion of chitosan or a derivative thereof is between at least 40% and 95.5% by weight.

4. System, according to claims 1 to 3, where the proportion of cyclodextrin or a derivative thereof with respect to the chitosan is between 0.5% and less than 60% by weight.

5. System, according to claims 1 to 4, where the degree of polymerisation of chitosan or the number of monomer units which form the chitosan or a derivative thereof is between 5 and 5,000, preferably between 30 and 600.

6. System, according to claims 1 to 5, where the chitosan or the derivative thereof has a molecular weight of between 1 and 2,000 kDa, preferably between 5 and 500 kDa, more preferably between 5 and 200 kDa.

7. System, according to claims 1 to 6, where the chitosan or the derivative thereof has a degree of deacetylation of between 30% and 95%, preferably between 50% and 95%.

8. System, according to claims 1 to 7, where the cyclodextrin is selected from natural cyclodextrins (alpha, beta or gamma), hydroxypropyl cyclodextrins, carboxymethylcyclodextrins, sulfobutylcyclodextrins, aminocyclodextrin, dimethylcyclodextrin, cyclodextrin phosphate, hydroxyethylcyclodextrin, acetyl-cyclodextrin, ethylcyclodextrins, trimethylcyclodextrins, carboxyethylcyclodextrin, glucosylcyclodextrin, 6-O-α-maltosylcyclodextrins, butyl-cyclodextrins, sulfated cyclodextrins, N,N-diethylaminoethylcyclodextrin, tert-butylsilylcyclodextrins, Silyl [(6-O-tert-butyldimethyl)-2,3,-di-O-acetyl)-cyclodextrins, Succinyl-(2-hydroxypropyl)-cyclodextrins, Succinyl-cyclodextrins, Sulfopropyl-cyclodextrins, polycyclodextrins.

9. System, according to claim 8, where the cyclodextrin is hydroxypropyl-α-cyclodextrin, hydroxypropyl-β-cyclodextrin, sulfobutylethyl-β-cyclodextrin or mixtures thereof.

10. System, according to claims 1 to 9, where the cyclodextrin exhibits a mean degree of substitution of between 4.2 and 7.

11. System, according to claims 1 to 10, which, in addition, comprises a biologically active molecule selected from the group formed by low-molecular-weight drugs, polysaccharides, proteins, peptides, lipids, oligonucleotides, nucleic acids and combinations thereof.

12. System, according to claims 1 to 11, where the biologically active molecule is a class 2, 3 or 4 drug, according to the definitions of the Biopharmaceutical Classification System adopted by the FDA; preferably, it is a class 4 drug.

13. System, according to claim 2, where the crosslinking agent is a polyphosphate salt, preferably sodium tripolyphosphate.

14. System, according to claims 1 to 13, where the mean size of the nanoparticles is between 1 and 999 nm, preferably between 100 and 800 nm.

15. System, according to claims 1 to 14, where the electric charge (Z potential) is between 0 and +60 mV measured in 1mM KCl.

16. A pharmaceutical composition which comprises a system such as that defined in any of claims 1 to 15 and a biologically active molecule capable of preventing, palliating or curing diseases.

17. Composition, according to claim 16, for administration by oral, buccal, sublingual, topical, transdermal, ocular, nasal, vaginal or parenteral route.

18. Composition, according to claims 16 and 17, where the biologically active molecule is selected from polysaccharides, proteins, peptides, lipids, nucleic acid-based molecules and combinations thereof.

19. Composition, according to claims 16 to 18, where the biologically active molecule is a class 2, 3 or 4 drug, according to the definitions of the Biopharmaceutical Classification System adopted by the FDA.

20. Composition, according to any of claims 16 to 18, where the biologically active molecule is triclosan, furosemide, insulin, heparin or molecules composed of nucleic acids.

21. Cosmetic composition which comprises a system such as that defined in any of claims 1 to 10 and 13 to 15 and a cosmetically active molecule.

22. Cosmetic composition, according to claim 21, where the cosmetically active molecule is selected from anti-acne agents, antifungal agents, antioxidant agents, deodorants, antiperspirants, anti-dandruff agents, skin whiteners, tanning lotions, UV-light absorbers, enzymes and cosmetic biocides.

23. A vaccine which comprises a system for the release of a biologically active molecule such as that defined in any of claims 1 to 15 and an antigen.

24. Vaccine, according to claim 23, where the antigen is selected from proteins, polysaccharides and DNA molecules.

25. Method for the obtainment of a system designed for the controlled release of a biologically active molecule, according to any of claims 1 to 15, which comprises:
a) preparation of a solution of chitosan or a derivative thereof in an aqueous medium or in a mixture of water with a polar solvent;
b) preparation of a solution of cyclodextrin or a derivative thereof in an aqueous medium or in a mixture of water with a polar solvent and, optionally, a crosslinking agent; and
c) mixing, under stirring, the solutions of steps a) and b) in such a way that the nanoparticles of chitosan-cyclodextrin are spontaneously obtained,
or, optionally:
a) preparation of a solution of chitosan or a derivative thereof and a cyclodextrin or a derivative thereof in an aqueous medium or in a mixture of water with a polar solvent;
b) preparation of a solution of the crosslinking agent in an aqueous medium or in a mixture of water with a polar solvent;
c) mixing, under stirring, the solutions of steps a) and b) in such a way that the nanoparticles of chitosan-cyclodextrin are spontaneously obtained.

26. Method for the obtainment of nanoparticles, according to claim 25, where the crosslinking agent is a tripolyphosphate, preferably sodium tripolyphosphate.

27. Method, according to any of claims 25 and 26, where the biologically active molecule is previously dissolved in steps a) or b) or in another aqueous or organic phase which is added to a) or b).

28. Method, according to claim 25, where the biologically active molecule is selected from polysaccharides, proteins, peptides, lipids, nucleic acid-based molecules and combinations thereof.

29. Method, according to claims 25 and 28, where the biologically active molecule is a class 2, 3 or 4 drug, according to the (Biopharmaceutical Classification System of the) FDA; preferably, it is a class 4 drug.

30. Method, according to claims 28 and 29, where the biologically active molecule is triclosan, furosemide, insulin, heparin or DNA plasmids.

31. Use of a system such as that described in claims 1 to 15 in the preparation of a gene therapy drug.
